# FASCICULE DE BREVET EUROPEEN

(11) **EP 1 242 805 B1**
(45) Date de publication et mention de la délivrance du brevet: **07.06.2006**
(21) Numéro de dépôt: 00993442.3
(22) Date de dépôt: 13.12.2000
(51) Int. Cl.: G01N 17/00

(54) **DISPOSITIF D'EXPOSITION A DES RAYONNEMENTS ELECTROMAGNETIQUES POUR TESTER LE VIEILLISSEMENT D'ECHANTILLONS**
PROBEBESTRAHLUNGSVORRICHTUNG DURCH ELEKTROMAGNETISCHE STRAHLUNGEN ZUR FESTSTELLUNG DES VERALTERUNGSZUSTANDS VON PROBEN
DEVICE FOR EXPOSING A SAMPLE TO ELECTROMAGNETIC RADIATION, FOR TESTING THE AGING OF SAMPLES

(30) Priorité: 13.12.1999 FR 9916038
(43) Date de publication de la demande: 25.09.2002
(73) Titulaire: Beraud, Michel, 83150 Bandol (FR)
(72) Inventeur: Beraud, Michel, 83150 Bandol (FR)
(74) Mandataire: Ballot, Paul Denis Jacques
(86) Numéro de dépôt international: PCT/FR2000/003499
(87) Numéro de publication internationale: WO 2001/044787

(56) Documents cités:
- EP-A- 0 320 209
- DE-A- 3 243 722
- US-A- 4 760 748

## Description

### 1. Domaine Technique

La présente invention concerne le domaine des dispositifs et appareils à source de rayonnements électromagnétiques pour tester le vieillissement accéléré d'échantillons sous l'effet de la lumière, ainsi que d'autres conditions atmosphériques, comme la température, l'humidité ou les précipitations d'eau... Les essais portent en particulier sur la tenue d'échantillons en matériaux polymères qui sont essentiellement sensibles aux rayonnements ultraviolets.

### 2. Etat de la Technique Antérieure

De façon connue, le vieillissement naturel des matériaux à la lumière solaire est simulé en exposant des échantillons à une source de rayonnements électromagnétiques dont la répartition spectrale doit être soigneusement choisie pour obtenir un vieillissement accéléré corrélable à celui constaté dans les conditions naturelles.

On connaît des dispositifs comprenant une rampe fixe de tubes à arc basse pression qui fournissent des rayonnements ultraviolets. Cette rampe de tubes parallèles est disposée en face des échantillons à tester.

L'inconvénient du rayonnement des tubes à arc basse pression est d'avoir une faible émissivité et un spectre ultraviolet très différent du spectre solaire, ce qui obère les essais.

Autre inconvénient, ces dispositifs ne permettent pas d'obtenir une exposition homogène des échantillons, si bien que les résultats d'essais ont une mauvaise reproductibilité.

On connaît des dispositifs utilisant des lampes à arc au Xénon, qui ont l'intérêt de présenter un spectre très proche du spectre solaire, lorsqu'elles sont filtrées convenablement.

Selon l'état des développements récents de la technique, on peut maintenant adopter de façon équivalente des lampes à vapeur de mercure moyenne pression filtrées, dont le spectre est très riche en rayonnements ultraviolets, et présente un bon équilibre entre les ultraviolets longs, de type U.V.A., et les ultraviolets courts, de type U.V.B., ce qui permet de bien reproduire le vieillissement dû à la lumière solaire.

On connaît des appareils d'essais de vieillissement dans lesquels les échantillons sont montés sur un porte-échantillon cylindrique vertical rotatif autour de lampes à arc au Xénon de forme tubulaire disposées verticalement.

La rotation des échantillons autour des lampes au Xénon, permet de régulariser l'exposition au rayonnement.

Cependant ces lampes présentent un fort dégagement thermique et il faut prévoir une circulation d'air en circuit ouvert ou en circuit fermé pour maintenir les échantillons à une température contrôlée.

Ces appareils ont l'inconvénient d'exposer les échantillons à des températures hétérogènes.

En effet, le circuit de ventilation comporte une zone d'aspiration d'air au dessus du porte-échantillons cylindrique qui provoque un flux d'air vertical comme dans une cheminée. Le flux d'air se réchauffe alors en progressant verticalement le long des lampes et des échantillons, si bien que les échantillons disposés en haut du porte-échantillons se trouvent nécessairement à une température supérieure à celle des échantillons disposés en bas. Des échantillons identiques situés à des hauteurs différentes sont donc soumis à des températures différentes, ce qui affecte encore la reproductibilité des essais. Dans la pratique certains utilisateurs sont contraints d'interrompre les essais pour intervertir les échantillons en cours de test, entre le haut et le bas du porte-échantillons.

On connaît aussi des dispositifs comportant une chambre d'essais parallèlépipédique avec des lampes au mercure disposées verticalement aux quatre coins de la chambre, autour d'un petit cylindre rotatif porte-échantillons disposé au centre de la chambre. Les lampes et la face externe des échantillons sont refroidies par une circulation d'air ventilé avec des arrivées d'air ouvertes dans les parois latérales de la chambre et une sortie d'air coiffant tout le cylindre porte-échantillons au plafond de la chambre.

Un inconvénient de ce dispositif est de présenter un encombrement excessif par rapport à la faible surface d'échantillons en test.

Ce dispositif a aussi l'inconvénient de présenter d'importantes pertes d'énergie lumineuse, puisque plus de 75% de la lumière ne se dirige pas directement vers le porte-échantillons.

Consécutivement, ce dispositif présente un mauvais rendement lorsqu'on rapporte l'amortissement du matériel ajouté aux dépenses énergétiques, à la surface d'échantillons en test, ce qui prétérite le coût des essais.

Il existe encore des appareils comportant une chambre d'essais rectangulaire dans laquelle est montée une cage porte-échantillons cylindrique, montée à rotation sur un arbre d'entraînement plein. Les échantillons sont longés par un flux d'air laminaire se propageant verticalement entre des ouvertures percées dans l'axe des génératrices du cylindre au plancher et au plafond de la chambre, dans le but d'isoler les échantillons du flux d'air chaud issu de la lampe et d'homogénéiser les températures d'essais.

Malgré le flux d'air laminaire, cet appareil a toujours l'inconvénient d'exposer les échantillons à des températures différentes, le flux d'air laminaire se réchauffant encore au contact des échantillons exposés au fort rayonnement lumineux. Dans la pratique, les échantillons disposés en haut de la cage sont ainsi exposés à une température supérieure de plusieurs degrés à celle des échantillons disposés en bas de la cage.

Le brevet EP-0 320 209 décrit ainsi une armoire de test atmosphérique comprenant un rack ou panier porte-échantillons rotatif autour d'un tube lumineux au Xenon disposé verticalement. Les échantillons sont refroidis par un flux d'air laminaire qui longe verticalement les parois internes des échantillons disposés le long des flancs droits du rack. Par économie, le flux d'air a un débit limité.

Cette armoire de test à flux d'air laminaire vertical à faible débit a l'inconvénient de ne pas apporter un refroidissement efficace des échantillons et d'induire des différences de température entre les échantillons disposés en bas et ceux disposés en haut du rack.

Le brevet US-4,760,748 décrit un autre appareil de test de vieillissement accéléré comprenant également un chassis cylindrique porte-échantillons monté en rotation autour d'un axe vertical et de tubes lumineux, avec un flux d'air laminaire ascendant refroidissant la face interne des échantillons. Le châssis cylindrique comporte des parois pleines percées de deux rangées d'ouvertures pour d'une part, disposer les échantillons, d'autre part former des orifices d'aspiration d'un flux d'air secondaire.

En fait, pour le refroidissement, l'appareil comporte un système de circulation d'air à deux flux, avec une colonne d'air principale ascendante, qui monte dans le cylindre porte-échantillons en longeant la face interne des échantillons et un flux d'air secondaire périphérique venant frapper la face externe des échantillons. Le flux d'air secondaire d'appoint provient d'une source d'air refroidi. Le flux d'air secondaire est créé par un effet d'aspiration provoqué au niveau des orifices du cylindre par l'ascension de la colonne d'air principale. Les orifices ont une obturation variable pour doser le flux d'air secondaire périphérique par rapport au flux d'air principal ascendant.

L'inconvénient de cet appareil est la complexité et l'encombrement particulièrement élevé de son système de circulation d'air à double flux.

Autre inconvénient, l'appareil présente un espace de test et une capacité en nombre d'échantillons bien restreints par rapport à l'encombrement volumineux de l'armoire avec son appareillage pour l'évacuation d'air en partie haute, la soufflerie et l'entraînement en rotation en partie basse, ainsi que le système de circulation d'air périphérique et la source d'air froid latérale.

Ce système de refroidissement à deux flux d'air a encore l'inconvénient d'induire des différences de température conséquentes entre la face interne irradiée et la face externe refroidie des échantillons.

On connaît par le document allemand DE-A-32 43 722, un dispositif bien différent d'essais de résistance à la lumière et aux intempéries comprenant une grande enceinte ventilée dans laquelle est disposé un ensemble horizontal comprenant une couronne de tubes lumineux insérées entre deux conduites d'air concentriques horizontales et un tambour porte-échantillons monté en rotation autour des conduites horizontales. Les deux conduites sont reliées à un ventilateur et communiquent entre elles ainsi qu'avec l'extérieur de l'enceinte de sorte que l'air extérieur est insufflé dans la première conduite puis retourne et s'insère dans l'espace entre la première et la seconde conduite pour longer les tubes lumineux en les refroidissant en revenant vers l'extérieur.

Quant aux échantillons disposés sur le tambour externe, ils sont refroidis séparément par un autre flux d'air vertical qui circule dans un circuit doté d'un autre ventilateur et d'un échangeur de refroidissement ainsi qu'éventuellement de moyens de chauffage.

Ce dispositif comporte donc deux systèmes de circulation d'air refroidissant d'une part les échantillons, d'autre part les tubes lumineux.

Dans ce dispositif, les tubes lumineux ainsi qu'un système d'aspersion sont disposés dans des conduites qui les séparent des échantillons, ce qui a l'inconvénient de nuire particulièrement à l'exposition lumineuse des échantillons et à l'efficacité du système.

Ce dispositif a encore l'inconvénient que le flux d'air vertical qui traverse le tambour porte-échantillons n'apporte pas un refroidissement efficace aux échantillons.

L'objet de la présente invention est de réaliser un dispositif d'exposition à des rayonnements pour tester le vieillissement d'échantillons permettant de soumettre les échantillons à une température et à une irradiation uniformes, sans les inconvénients précités.

L'invention a en particulier pour objectif d'assurer une ventilation efficace des échantillons lors des tests effectués avec le dispositif.

Un autre objectif de l'invention est d'obtenir un dispositif présentant un nombre de lampe et un encombrement réduits.

Un autre objectif parallèle de l'invention est d'obtenir un dispositif de conception simple et de rendement lumineux élevé rapporté à la surface d'échantillons exposés de façon à réduire le coût des essais.

Enfin, l'invention a aussi pour objectif de faciliter la manipulation des échantillons préliminaire aux tests de vieillissement.

### EXPOSE SOMMAIRE DE L'INVENTION

Succinctement ces objectifs sont atteints selon l'invention, en réalisant un dispositif comportant une cage porte-échantillons rotative dont l'axe de rotation est disposé horizontalement, de sorte que les échantillons se trouvent cycliquement dans la partie supérieure, puis dans la partie inférieure de l'enceinte, ce qui permet de s'abstraire des différences de températures inhérentes, et plus essentiellement en prévoyant que le dispositif présente un flux d'air tourbillonnaire autour de l'axe de la cage.

Par flux d'air tourbillonnaire, on entend dans la présente, désigner l'écoulement d'une masse d'air animée d'une part d'un mouvement de rotation et de convergence autour d'un axe et d'autre part, d'un mouvement de translation dirigé sensiblement dans cet axe. Un flux d'air tourbillonnaire se caractérise aussi par un mouvement giratoire, circulaire, hélicoïdal ou spiral de l'air autour d'un axe avec une vitesse inversement proportionnelle à la distance par rapport à l'axe.

Dans le dispositif selon l'invention, le flux d'air entre à la périphérie de la cage en arrivant transversalement à l'axe et en ayant essentiellement des composantes tangentielles et/ou radiales à la périphérie de la cage, puis s'évacue au centre de la cage parallèlement à l'axe, en ayant essentiellement une composante axiale dans la partie centrale de la cage, ce qui assure avantageusement une symétrie et une régularité du flux d'air.

On peut aisément constater la présence d'un flux tourbillonnaire à l'aide d'un fumigène, ce qui fait apparaître un enroulement de volute de fumée pouvant tourner plusieurs fois à la périphérie de la cage, ce qui optimise avantageusement les échanges caloriques au niveau des échantillons, avant de s'évacuer rapidement dans l'axe de la cage porte-échantillons. Le flux d'air peut ainsi tournoyer autour d'une dépression en formant une sorte d'entonnoir ou de vortex.

L'invention est réalisée avec un dispositif d'exposition à des rayonnements pour tester le vieillissement d'échantillons, ayant un enceinte et comprenant :
- une cage porte-échantillons rotative autour d'un axe horizontal, et
- des moyens pour positionner fixement et alimenter au moins une lampe à rayonnement électromagnétique, dans la partie centrale de la cage,
   avec la particularité qu'il comprend un système de circulation d'air apte à générer un flux d'air tourbillonnaire autour de l'axe de la cage, le système comprenant des ouvertures fixes de passage d'air disposées autour de la périphérie de la cage rotative, et un orifice de passage d'air disposé dans l'axe de la cage.

Le dispositif comprend un système de circulation d'air avec des arrivées d'air à la périphérie de la cage et une évacuation d'air dans l'axe de la cage.

De préférence, le système de circulation d'air comprend des ouvertures fixes d'arrivée d'air disposées régulièrement à la périphérie de la cage avec une symétrie de révolution autour de l'axe, chaque ouverture étant orientée dans une direction comprise entre la direction radiale et la direction tangentielle à la périphérie de la cage, et une conduite d'évacuation d'air disposée dans le prolongement axial de la partie centrale de la cage, la conduite étant orientée dans une direction parallèle à l'axe.

De préférence, la cage est montée en rotation sur un moyeu évidé axialement, le flux d'air s'évacuant dans l'évidement axial du moyeu.

De préférence, le système de circulation d'air comporte des moyens d'aspiration d'air disposés dans la lumière d'évidement du moyeu.

De préférence, le système de circulation d'air est en circuit ouvert, ce qui permet avantageusement d'aspirer directement l'air frais du laboratoire.

Alternativement, le système de circulation d'air peut fonctionner en circuit fermé.

Selon le mode de réalisation préféré de l'invention, l'enceinte cylindrique comporte des ouïes d'entrée d'air disposées sensiblement à la périphérie de la cage. Il est prévu avantageusement que les ouïes d'entrée d'air périphériques de l'enceinte sont dirigées non-radialement et que chaque ouïe d'entrée d'air périphérique a une profondeur, une ouverture et un angle d'inclinaison par rapport à la direction radiale, tels que le rayonnement de chaque lampe positionnée dans la partie centrale de la cage ne traverse pas l'ouïe et ne s'échappe pas directement de l'enceinte.

Selon le mode de réalisation préféré de l'invention, la cage comporte des éléments centraux et des éléments périphériques s'étendant dans la direction axiale et reliés par des éléments radiaux, les éléments centraux et périphériques s'étendant dans un seul demi-espace délimité par les éléments radiaux.

Selon le mode de réalisation préféré, la cage comporte des éléments circulaires de diamètres différents permettant de disposer des échantillons sous des angles d'exposition différents.

Selon une alternative avantageuse, les éléments centraux de la cage se développent en hélices autour de l'axe.

Selon le mode de réalisation préféré de l'invention; la cage est reliée au moyeu par un mécanisme d'entraînement débrayable, permettant une rotation libre de la cage.

De façon avantageuse, le mécanisme comporte des moyens de débrayage en rotation sous l'effet d'un couple axial limite entre la cage et le moyeu.

De façon avantageuse, le mécanisme comporte des moyens de débrayage ou de dé-solidarisation en translation sous l'effet d'un effort axial limite entre la cage et le moyeu.

Selon une alternative de réalisation, le disposif comporte un réservoir d'eau disposé pour baigner une partie de la cage

Enfin, selon l'invention, le dispositif est destiné à fonctionner avec l'axe de la cage rotative disposé dans une direction horizontale.

L'invention porte également sur un procédé de test de vieillissement d'échantillons ayant la particularité de mettre en oeuvre un tel dispositif d'exposition à des rayonnements. le procédé comporte les étapes décrites dans la revendication 20.

L'invention peut aussi être réalisée avec un procédé de test de vieillissement d'échantillons comportant des étapes consistant à:
- soumettre les échantillons à une rotation verticale autour d'une ou de lampes à rayonnement électromagnétique disposées sur ou autour de l'axe de rotation horizontal, et
- générer un flux d'air tourbillonnaire autour de l'axe de rotation des échantillons, le flux d'air ayant essentiellement des composantes perpendiculaires à l'axe de rotation, au niveau des échantillons.

### BREVE DESCRIPTION DES DESSINS

D'autres objectifs, caractéristiques et avantages de l'invention apparaîtront à la lecture de la description ci-après d'un mode de réalisation, donné uniquement à titre d'exemple non-limitatif, description intégrant les dessins annexés sur lesquels :
- la figure 1A représente schématiquement une vue en coupe axiale de dispositif d'exposition à des rayonnements selon l'invention,
- la figure 1B représente schématiquement une vue de face en coupe transversale du dispositif selon l'invention,
- Les figures 2B, 2A et 2C représentent des vues d'une réalisation de dispositif selon l'invention, avec capot escamoté, la figure 2A montrant le dispositif sans capot et sans porte-lampe, vu en coupe axiale, la figure 2B montrant le capot escamoté, en vue de trois quart vers l'intérieur, la figure 2C montrant la chaîne cinématique du dispositif, vue en coupe transversale vers l'arrière suivant le tracé C-C de la figure 2A.
- les figures 3A et 3B représentent une vue de profil et une coupe transversale de détails d'une réalisation avantageuse de capot pour dispositif selon l'invention,
- les figures 4A et 4B représentent une coupe axiale et une vue de face d'une réalisation avantageuse de cage porte-échantillons pour dispositif selon l'invention,
- la figure 5A représente une vue en demi-coupe axiale et en demi-profil d'une réalisation avantageuse de mécanisme d'entraînement débrayable pour cage porte-échantillons de dispositif selon l'invention, et
- la figure 5B représente une vue en demi-coupe transversale et de face du mécanisme d'entraînement débrayable de la figure 5A.

### DESCRIPTION DETAILLEE DU MODE DE REALISATION PREFERE

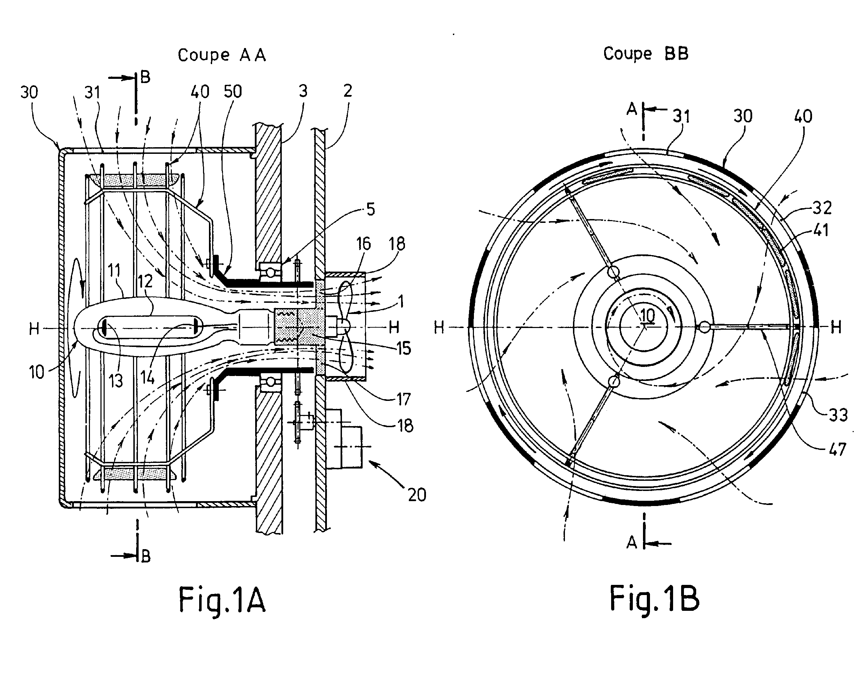

Sur les figures 1 à 5, il apparaît clairement que le dispositif selon l'invention est, de préférence, globalement circulaire et symétrique de révolution autour d'un axe H-H destiné à être disposé dans une direction horizontale.

Dans la description du mode de réalisation et de fonctionnement ci-après, on considérera pour simplifier que le dispositif est symétrique de révolution autour de l'axe H-H, des géométries plus complexes pouvant être envisagées par l'homme du métier sans sortir du cadre de la présente invention.

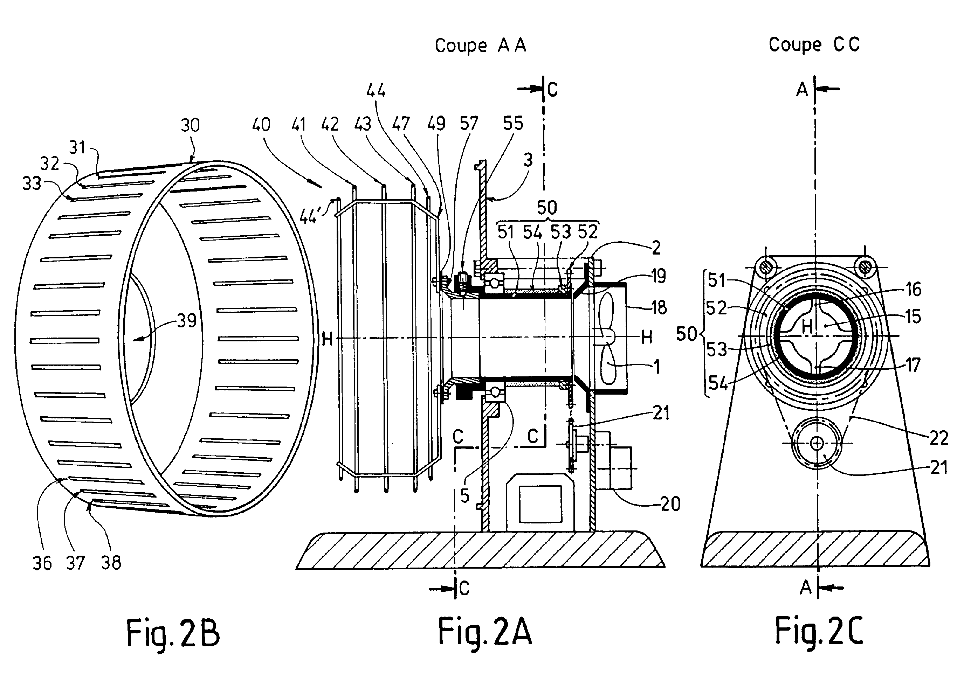

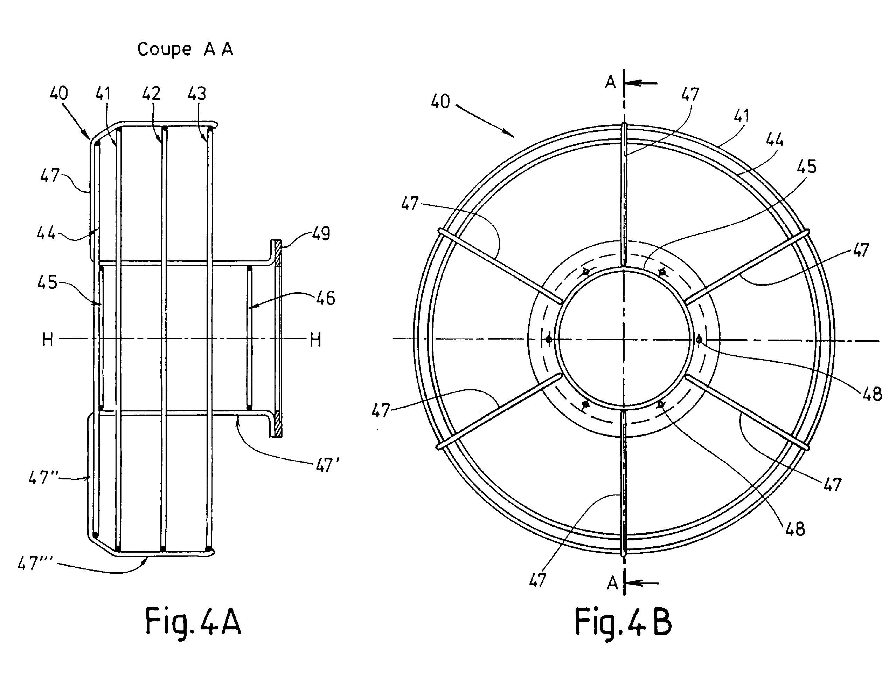

La figure 1A montre ainsi que le dispositif selon l'invention comporte une enceinte 3, 30 cylindrique et circulaire. Alternativement, le dispositif peut comporter une enceinte cylindrique mais de section carrée, rectangulaire ou autre. L'enceinte comporte un capot 30 amovible de préférence en forme de cuve, qui vient se fixer hermétiquement sur une platine 3 fixe. L'enceinte 3, 30 contient une cage porte-échantillons 40 de forme circulaire qui peut être réalisée selon l'exemple des figures 2 et 4, à partir d'une armature de tiges métalliques 41 à 47 cintrées en forme d'éléments circulaires 41,42,43,44,45,46 et d'éléments recourbés 47 disposés en quartiers et solidarisés entre eux.

La cage 40 est solidarisée, par l'intermédiaire d'une pièce d'entraînement circulaire 49 et d'un mécanisme débrayable 55,56,57,58,59 avec un moyeu 50-51.

Le moyeu 50-51 est monté en rotation, éventuellement sur roulement 5 à billes ou à rouleaux, sur la ou les platines 2-3 qui offrent un support fixe.

Le moyeu 50 est entraîné en rotation par une chaîne cinématique comportant un moteur désaxé 20 relié par une transmission 21, 22 à engrenage ou à chaîne.

Selon le mode de réalisation préféré de l'invention, le moyeu 50-51 est complètement évidé axialement, l'évidement formant une conduite d'évacuation de flux d'air.

De même selon le mode de réalisation préféré, la partie centrale de la cage porte-échantillons 40 est évidée, aucune tige de l'armature n'apparaissant dans la partie centrale de la cage qui correspond à la prolongation de la partie évidée du moyeu 51.

En effet, l'invention prévoit de disposer fixement une ou des lampes 10 à rayonnement électromagnétique dans la partie centrale de la cage rotative 40.

La ou les lampes 10 sont raccordées, c'est-à-dire fixées et alimentées électriquement, directement par l'intermédiaire d'une pièce axiale 15 ayant des branches radiales 16, 17 en forme d'aster, à la platine 2 de support fixe, ou indirectement par l'intermédiaire du corps 18 du ventilateur 1.

Si une seule lampe à ultraviolet 10 équipe le dispositif selon l'invention, il est préférable que la lampe 10, généralement tubulaire ou symétrique de révolution soit positionnée dans la partie centrale de la cage, l'axe de la lampe étant disposé dans l'axe H-H de la cage.

Si deux ou plusieurs lampes équipent le dispositif selon l'invention, il est préférable que les lampes soient disposées symétriquement autour de l'axe de la cage et parallèlement audit axe H-H.

De façon particulièrement avantageuse, la disposition de la ou des lampes 10 dans la partie centrale de la cage porte-échantillons 40 sur ou autour de l'axe de rotation H-H, permet d'exposer les échantillons en rotation à un flux de lumière, parfaitement homogène (à condition que les échantillons soient disposés parallèlement et à la même distance radiale de l'axe).

Il est encore prévu, selon le mode de réalisation préféré de l'invention, que le dispositif comporte des moyens d'aspiration d'air 1 disposés dans le prolongement de l'évidement du moyeu 50.

Il est prévu par exemple qu'un ventilateur 1 est disposé dans le prolongement arrière de l'évidement du moyeu 50.

Le ventilateur 1 peut être fixé sur la platine arrière 2 fixe.

De façon avantageuse, les parties centrales de la cage 40 et du moyeu 50, ainsi que leurs prolongements sont complètement dégagés, à l'exception du porte-lampe 15, pour laisser passer librement le flux d'air.

Ainsi, selon l'invention l'évacuation du flux d'air se fait dans la direction axiale H-H au centre de la cage 40, le dispositif comportant avantageusement une cage 40 et un moyeu 50 dont la partie centrale est complètement évidée.

D'autre part, l'invention prévoit de disposer des arrivées d'air 31,32,33,...,36,37,38 à la périphérie de la cage 40.

Selon le mode de réalisation préféré de l'invention, l'enceinte cylindrique 30 comporte ainsi des ouïes 31 à 38, c'est-à-dire des ouvertures d'entrée d'air, percées sur le pourtour cylindrique ou quasi-cylindrique (légèrement conique), du capot 30 ou plus généralement de l'enceinte.

Lors des tests, il est prévu de disposer les échantillons à la périphérie de la cage, en les solidarisant sur les éléments circulaires 41,42,43 de l'armature de la cage, notamment à l'aide de pinces.

On considère communément que les échantillons sont découpés en plaques ou en cartes, de forme planaire en général, si bien que les échantillons viennent occuper des surfaces sécantes ou tangentielles à la périphérie de la cage circulaire 40, comme illustré figure 1B.

En fonctionnement, les échantillons sont donc en position tangentielle ou sécante à la périphérie de la cage 40, la cage est mise en rotation et les moyens 1 d'aspiration d'air amorcent un flux d'évacuation d'air dans l'axe H-H de la cage 40, de la lampe 10 et du moyeu 50.

De façon particulièrement avantageuse, l'air réchauffé par la ou les lampes 10 en position axiale est évacué directement sans échauffer les échantillons.

La chaleur de convection dégagée par les lampes en fonctionnement ne perturbe donc pas la température des échantillons en cours de test.

D'autre part, l'évacuation d'air provoque un flux d'entrée d'air à la périphérie de l'enceinte 30 qui vient frapper les échantillons orthogonalement ou avec un angle d'incidence par rapport à leur plan, comme illustré figure 1B.

De façon avantageuse, l'incidence du flux d'air sur les échantillons améliore les échanges thermiques.

Contrairement aux dispositifs de l'état de la technique, les échantillons ne sont donc pas longés par un flux d'air laminaire, mais sont soumis à un flux d'air incident et tourbillonnaire.

Dans le cadre de certaines réalisations, le flux d'entrée d'air peut être strictement radial au niveau des échantillons disposés à la périphérie de la cage 40.

Ce cas se présente en particulier lorsque chaque ouïe d'aspiration d'air 31, 32, 33,... est percée radialement dans la partie cylindrique de l'enceinte 30 en supposant que la rotation de la cage 40 ne modifie pas la circulation d'air.

Dans le cadre d'autres réalisations, le flux d'entrée d'air à la périphérie de la cage porte-échantillons 40, comporte à la fois une composante radiale et une composante tangentielle, si bien que les échantillons sont soumis à un flux d'air ayant un certain angle d'incidence.

Dans le mode de réalisation préféré, il est ainsi prévu de percer des ouïes non-radiales 31 à 38 à la périphérie de l'enceinte cylindrique 30.

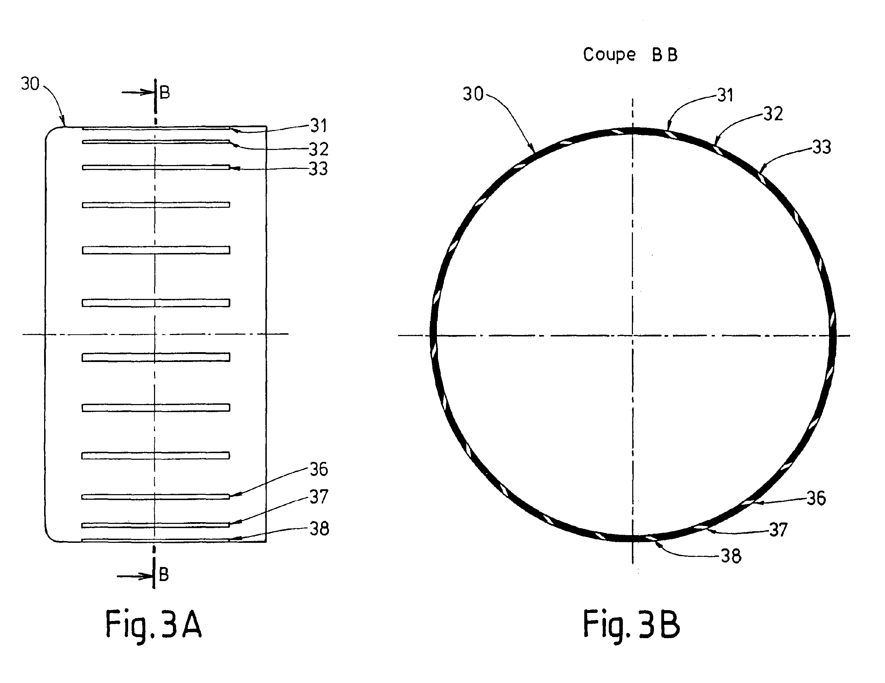

Les figures 3A et 3B montrent ainsi que le pourtour quasi-cylindrique du capot 30 de l'enceinte est entaillé par de fines ouvertures 31,32,33,...,36,37,38 formant des fentes tracées axialement, ces fentes ayant la particularité de ne pas s'enfoncer suivant un plan radial, mais suivant une direction oblique par rapport à la direction radiale ; à la limite les ouvertures 31 à 38 peuvent être quasiment tangentielles au cylindre du capot 30.

De façon particulièrement avantageuse, les ouïes obliques 31 à 38 permettent au flux d'entrée d'air d'avoir des composantes tangentielles et radiales au niveau des échantillons disposés à la périphérie de la cage 40.

La circulation d'air provoque ainsi un flux d'air tourbillonnaire autour de l'axe H-H de la cage 40, le flux d'air n'ayant pratiquement pas de composante axiale mais uniquement des composantes tangentielles et/ou radiales à la périphérie de la cage 40, alors que le flux d'air a uniquement une composante axiale H-H au centre de la cage 40.

Dans la présente, l'expression "flux d'air tourbillonnaire" englobe le cas limite dans lequel le flux d'air à la périphérie de la cage présente uniquement une composante radiale, ainsi que l'autre cas limite dans lequel le flux d'air à la périphérie de la cage présente uniquement une composante tangentielle.

La circulation d'air est ainsi avantageusement analogue à un mouvement de siphon autour de l'axe de la cage, ce qui assure des échanges thermiques optimums au niveau des échantillons.

Un autre avantage essentiel de la disposition oblique des ouïes d'entrée d'air 31 à 38 à la périphérie de l'enceinte cylindrique 30 est d'obturer la sortie directe des rayonnements de la lampe 10 tout en permettant le passage de l'air de façon analogue au voletage d'un store ou d'un volet.

Dans le mode de réalisation préféré de l'invention, chaque fente d'ouïe 31 a une ouverture réduite, mais un angle d'inclinaison élevé par rapport à la direction radiale ainsi qu'une profondeur élevée, de sorte que le rayonnement direct de chaque lampe 10 positionnée dans la partie centrale de la cage ne traverse pas l'ouïe 31 et ne s'échappe pas de l'enceinte.

D'autre part, il est prévu optionnellement, comme illustré sur la figure 2A, que le capot 30 comporte un hublot central 39 en verre anti-U.V..

Ce hublot 39 dont le verre coupe les rayonnements ultraviolets permet avantageusement d'observer les échantillons en cours de test, en évitant les rayonnements nocifs de la lampe.

De façon avantageuse, la partie centrale du hublot 39 peut être masquée par une pièce centrale fixée par des branches radiales en forme d'aster (non illustré).

Le capot 30 est réalisé de préférence en tôle, par emboutissage, ce qui donne un capot de forme légèrement conique, quasi-cylindrique.

Une telle réalisation légère a l'avantage de limiter le prix de l'équipement et des investissements de tests de vieillissement.

Les figures 4A et 4B montrent que la cage 40 est réalisée de préférence à partir de fils ou de tiges métalliques semi-rigides 41 à 47.

Le principal avantage d'une telle réalisation est de limiter le poids de la cage 40, donc le dimensionnement du moteur d'entraînement 20.

Une telle réalisation facilite également la circulation d'air et la manipulation des échantillons.

Cette réalisation légère a aussi l'avantage de limiter le coût de l'équipement.

Dans une première forme de réalisation simplifiée, la cage 40 est formée d'éléments circulaires 41,42,43 de diamètre identique, solidarisés par des éléments rayonnants 47 arqués comme dans un panier ou une roue d'écureuil. Les éléments rayonnants 47 comportent simplement une portion parallèle à l'axe pour fixer les éléments circulaires 41,42,43 et une portion radiale pour venir se fixer sur une rondelle 49 de diamètre médian, solidaire du moyeu d'entraînement 50.

Dans une autre forme de réalisation, illustrée sur les figures 1A et 2A, la cage comporte plusieurs éléments circulaires 41,42,43 et 44-44' de diamètres différents pour pouvoir positionner des échantillons plans soit parfaitement parallèlement à l'axe en les fixant par exemple aux éléments 41,42,43 de même diamètre, soit en position oblique par rapport à l'axe en les fixant par exemple aux éléments 41 et 44 de diamètres différents.

De façon avantageuse, cette armature multi-position permet de disposer des échantillons de longueurs différentes sous des angles différents d'incidence de rayonnement et à des distances différentes de la source lumineuse, ce qui permet d'exposer des échantillons de longueurs différentes à la même puissance surfacique lumineuse.

Dans la forme de réalisation préférée, illustrée sur la figure 4, la cage 40 a une armature particulière en forme de parapluie.

L'armature est formée d'éléments recourbés 47 disposés en quartier suivant des plans radiaux de façon à rayonner autour de la partie centrale évidée de la cage 40.

Comme le montre la figure 4A, chaque élément recourbé 47 comporte une portion ou élément central 47' s'étendant parallèlement à l'axe H-H à la limite de la partie centrale évidée de la cage 40. L'élément central 47' est relié à une portion ou élément 47" s'étendant dans la direction radiale à l'opposé du centre. Cet élément radial 47" est lui-même relié ou prolongé par un élément périphérique 47"' parallèle à l'axe, qui délimite la périphérie de la cage 40.

La particularité de l'armature de cage 40 de la figure 4 est que la portion centrale 47' et la portion périphérique 47"' de chaque élément recourbé 47 sont situées dans le même demi-espace délimité par le plan des portions radiales 47"'. Chaque élément recourbé 47 a donc une forme de crochet.

Par la suite, les éléments circulaires 41,42,43,44 sont fixés, notamment par soudure, sur les portions périphériques 47"' des éléments recourbés 47 pour former l'armature de la cage circulaire 40. De même, de petits éléments circulaires 45 et 46 peuvent être solidarisés avec les portions centrales 47' des éléments recourbés 47 pour achever la formation de l'armature et augmenter avantageusement la rigidité de la cage circulaire 40.

Enfin, les portions centrales 47' de l'armature de la cage sont fixées par des attaches, par soudure, ou tout autre moyen, sur une pièce en forme de rondelle 49 destinée à être solidaire du moyeu 50.

Une telle armature de cage en parapluie permet avantageusement de disposer des instruments fixement au sein de la cage rotative (disposition non-illustrée).

Les instruments, de type capteurs ou actionneurs, sont fixés à la platine 3 de support fixe du dispositif et font saillie de la platine (parallèlement ou obliquement par rapport à l'axe) pour s'engager au sein de la cage rotative 40 sans affecter la rotation.

Par exemple (non-illustré), il est prévu avantageusement de fixer une sonde de température à la platine 3, au-dessus de la lampe axiale 10, l'extrémité active de la sonde s'avançant entre la lampe et les échantillons sans empêcher ainsi la rotation de la cage.

Autre exemple (non-illustré), il est prévu de disposer une rampe d'aspersion d'eau en dessous et parallèlement à la lampe axiale 10 en la fixant à la platine 3 pour la relier à une arrivée d'eau.

De façon avantageuse, la rampe est ainsi immobilisée au sein de la cage rotative parallèlement au plan des échantillons, qui sont alors aspergés régulièrement sur toute leur surface à chaque cycle de rotation.

En outre, selon une forme de réalisation améliorée non illustrée, il est prévu que les portions centrales (47') des éléments recourbés qui s'étendent dans la direction axiale H-H ne sont pas rectilignes mais s'enroulent en hélices autour de l'axe H-H de la cage 40.

L'avantage d'une telle amélioration est d'éviter qu'un échantillon soit partiellement plongé dans l'ombre de la portion centrale d'un élément recourbé. Le rayonnement électromagnétique de la lampe centrale atteint ainsi uniformément chaque échantillon, quelle que soit sa position à la périphérie de la cage.

Par ailleurs, il est prévu selon une option avantageuse que le dispositif selon l'invention comporte un réservoir d'eau baignant la portion inférieure de la cage rotative.

Un bassin en forme de demi-lune (non-illustré), peut ainsi être fixé au bas de la platine 3 du dispositif, la cage 40 étant plongée partiellement dans l'eau remplissant le bassin.

Une telle disposition permet de tester la tenue des échantillons au vieillissement sous l'action combinée de l'humidité, de l'immersion dans l'eau, et des rayonnements électromagnétiques comme les ultraviolets, ce qui rassemble avantageusement la plupart des agents naturels de vieillissement.

Maintenant du point de vue mécanique, la cage 40 est entraînée en rotation par une chaîne cinématique 50 qui comporte des dispositions particulières, comme illustré sur les figures 2A et 2C.

Il est prévu avantageusement que le moteur d'entraînement 20 est désaxé au lieu d'être situé dans l'axe H-H de la cage 40.

Ceci permet de dégager complètement la portion centrale évidée du dispositif et son prolongement, pour ne pas gêner la circulation d'air.

Le moteur 20 est fixé à une platine 2 de support fixe. Le pignon de sortie 21 du moteur entraîne une couronne ou un plateau denté 52 fixé au moyeu 50-51.

La transmission peut se faire soit directement par engrenage, le pignon 21 et le plateau 52 dentés étant en contact, soit indirectement par l'intermédiaire d'une chaîne, ou d'une courroie 22 tendue entre un pignon 21 et une couronne 52 à gorge, comme illustré figure 2C, ou encore par tout autre moyen de transmission.

Dans l'exemple de réalisation des figures 2A et 5A, la couronne ou le plateau d'entraînement 52 est attaché par des vis sur un écrou 53 bloqué sur le pas de vis du moyeu 51.

L'écrou 53 permet en outre de bloquer une entretoise 54 cylindrique contre la bague du roulement à billes 5 ou le pourtour circulaire de la platine 3.

Comme l'autre extrémité 55 du moyeu est évasée, le moyeu 51 est alors immobilisé à rotation par rapport à la platine 3 de support fixe.

Enfin, de façon particulièrement avantageuse, il est prévu que la cage 40 est reliée au moyeu 51 par l'intermédiaire d'un mécanisme débrayable 50.

Le moyeu 50 est ainsi constitué de deux corps concentriques 57 et 51 à extrémités évasées 59 et 55, emboîtés l'un dans l'autre.

Comme l'illustre la figure 5A, le corps 57 du moyeu 50 a une forme évasée en entonnoir s'achevant par une extrémité 59 circulaire plane, creusée de pas de vis 58 pour fixer la cage 40 par l'intermédiaire de la rondelle 49.

L'autre extrémité, cylindrique, du corps 57 du moyeu 50 est creusée d'une gorge annulaire 56 sur tout le pourtour.

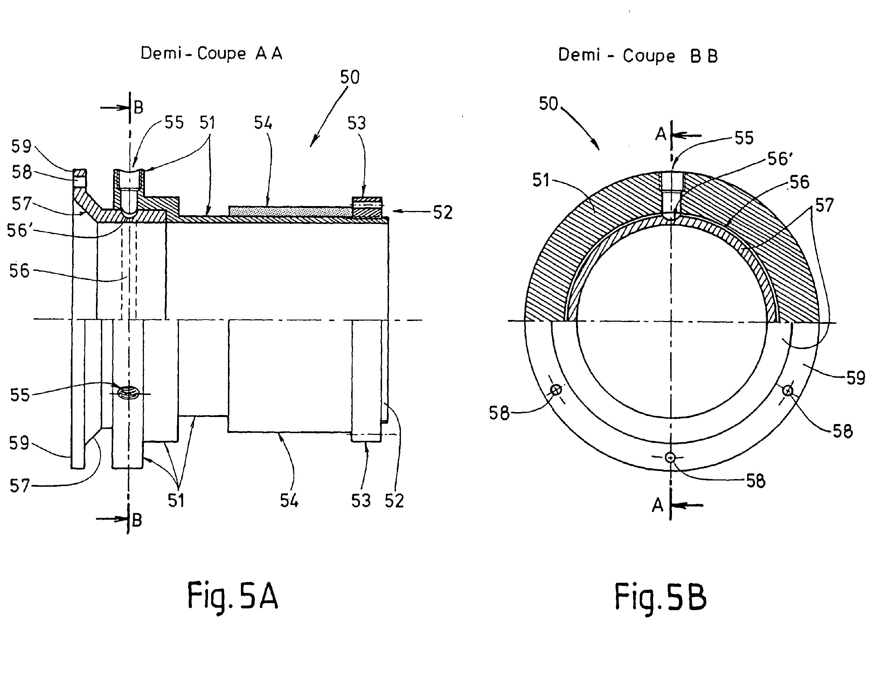

En outre, la gorge annulaire 56 est creusée de plusieurs cavités hémisphériques 56' en plusieurs points de son pourtour pour approfondir la depression de la gorge 56. Selon l'exemple de la figure 5, il est prévu ainsi de disposer trois cavités 56' de façon équidistante, à cent-vingts degrés l'une de l'autre, sur le pourtour de la gorge annulaire 56.

Corrélativement, l'extrémité évasée du corps principal 51 du moyeu 50 est percée radialement de logements tubulaires 55, correspondant aux cavités 56' d'approfondissement de la gorge 56 du corps d'extrémité 57.

Chaque logement 55 est destiné à recevoir une bille et un ressort maintenus par une vis de réglage en pression.

Lorsque le corps d'extrémité 57 est emboîté dans le corps principal 51 du moyeu 50, chaque bille vient donc s'engager dans la gorge annulaire 56, puis se bloque au fond de la cavité hémisphérique 56' correspondante.

Comme les billes sont maintenues au fond des cavités 56' creusées dans la gorge 56, ce mécanisme permet de bloquer en translation et en rotation le corps 57 par rapport au corps 51 du moyeu, de façon analogue au clavetage d'un barillet dans un cylindre de serrure.

Cependant si un couple excessif est exercé sur le corps 57 par rapport au corps 51 du moyeu 50, les billes sont repoussées hors de leurs cavités 56' respectives et le corps 57 se met à tourner librement autour du corps 51, les billes restant engagées dans la gorge annulaire 56.

Ce mécanisme permet très avantageusement un débrayage en rotation de la cage 40 par rapport au moyeu 50, lorsqu'il apparaît un couple axial excessif entre la cage et le moyeu.

De façon avantageuse, ce mécanisme débrayable permet à un opérateur de tourner la cage 40, alors que le moteur 20 est à l'arrêt.

De même, ce mécanisme évite une atteinte du moteur 20 lorsque la cage 40 se bloque accidentellement en rotation.

En outre, ce mécanisme permet très avantageusement un découplage en translation entre la cage 40 et le moyeu 50 lorsqu'on effectue une traction axiale excessive sur la cage.

En effet, en exerçant une traction sur le corps d'extrémité 57 dans la direction axiale, vers l'avant, les billes finissent par s'échapper du lit de la gorge annulaire 56 et le corps d'extrémité 57 se désolidarise du corps principal 51 du moyeu 50.

Ce mécanisme permet donc d'escamoter ou de débrayer en translation la cage du dispositif.

Enfin, il est prévu avantageusement que chaque logement 55 comporte une extrémité resserrée à un diamètre inférieur au diamètre des billes, pour éviter aux billes de s'échapper des logements 55 lorsque le corps 57 est désolidarisé.

D'autres modes de réalisation, variantes et améliorations pourront être mises en oeuvre par l'homme de métier sans sortir du cadre de la présente invention, l'objet de la protection étant défini dans les revendications ci-après.

## Revendications

1. Dispositif d'exposition à des rayonnements pour tester le vieillissement d'échantillons, ayant une enceinte (30) et comprenant :
- une cage (40) porte-échantillons rotative autour d'un axe horizontal (HH), et
- des moyens (15) pour positionner fixement et alimenter au moins une lampe (10) à rayonnement électromagnétique, dans la partie centrale de la cage (40),
**caractérisé en ce qu'**il comprend un système de circulation d'air apte à générer un flux d'air tourbillonnaire autour de l'axe (H-H) de la cage, le système comprenant des ouvertures (31,32,33) fixes de passage d'air disposées autour de la périphérie (41) de la cage (40) rotative, et un orifice (50) de passage d'air disposé dans l'axe (H-H) de la cage.

2. Dispositif selon la revendication 1, **caractérisé en ce que** le système de circulation d'air comprend des ouvertures fixes (31-38) d'arrivée d'air disposées régulièrement à la périphérie (41) de la cage (40) avec une symétrie de révolution autour de l'axe, chaque ouverture (31,32,33,...,38) étant orientée dans une direction comprise entre la direction radiale et la direction tangentielle à la périphérie de la cage, et une conduite (50) d'évacuation d'air disposée dans le prolongement axial (H) de la partie centrale de la cage (40), la conduite (50) étant orientée dans une direction parallèle à l'axe (H-H).

3. Dispositif selon la revendication 1 ou 2, **caractérisé en ce que** la cage (40) est montée en rotation sur un moyeu (50) évidé axialement (H-H), le flux d'air s'évacuant dans l'évidement axial du moyeu.

4. Dispositif selon la revendication 3, **caractérisé en ce que** le système de circulation d'air comporte des moyens (1) d'aspiration d'air disposés dans la lumière d'évidement du moyeu.

5. Dispositif selon l'une des revendications 1 à 4, **caractérisé en ce que** le système de circulation d'air est en circuit ouvert.

6. Dispositif selon l'une des revendications 1 à 5, **caractérisé en ce que** l'enceinte cylindrique (30) comporte des ouïes (31-38) d'entrée d'air disposées sensiblement à la périphérie de la cage (40).

7. Dispositif selon la revendication 6, **caractérisé en ce que** les ouïes (31-38) d'entrée d'air périphériques de l'enceinte (30) sont dirigées non-radialement.

8. Dispositif selon la revendication 7, **caractérisé en ce que** chaque ouïe (31) d'entrée d'air périphérique a une profondeur, une ouverture et un angle d'inclinaison par rapport à la direction radiale, tels que le rayonnement de chaque lampe (10) positionnée dans la partie centrale de la cage (40) ne traverse pas l'ouïe (31) et ne s'échappe pas directement de l'enceinte (30).

9. Dispositif selon l'une des revendications 1 à 8, comprenant des moyens (15) pour positionner axialement une lampe (10) à rayonnement électromagnétique sur l'axe (H-H) de la cage.

10. Dispositif selon l'une des revendications 1 à 9, comprenant des moyens pour positionner symétriquement des lampes à rayonnement électromagnétique autour de l'axe de la cage.

11. Dispositif selon l'une des revendications 1 à 10, **caractérisé en ce que** la cage (40) comporte des éléments centraux (47') et des éléments périphériques (47"') s'étendant dans la direction axiale (H-H) et reliés par des éléments radiaux (47"), les éléments centraux et périphériques (47',47"') s'étendant dans un seul demi-espace délimité par les éléments radiaux (47").

12. Dispositif selon l'une des revendications 1 à 11, **caractérisé en ce que** la cage comporte des éléments circulaires (41,44,44') de diamètres différents permettant de disposer des échantillons sous des angles d'exposition différents.

13. Dispositif selon la revendication 11 ou 12, **caractérisé en ce que** les éléments centraux de la cage se développent en hélices autour de l'axe.

14. Dispositif selon l'une des revendications 3 à 13, **caractérisé en ce que** la cage (40) est reliée au moyeu (50) par un mécanisme d'entraînement débrayable (51,55,56,57) permettant une rotation libre de la cage.

15. Dispositif selon la revendication 14, **caractérisé en ce que** le mécanisme comporte des moyens de débrayage en rotation sous l'effet d'un couple axial limite entre la cage et le moyeu.

16. Dispositif selon la revendication 14 ou 15, **caractérisé en ce que** le mécanisme comporte des moyens de débrayage ou de dé-solidarisation en translation sous l'effet d'un effort axial limite entre la cage et le moyeu.

17. Dispositif selon l'une des revendications 1 à 16, **caractérisé en ce qu'**il comporte un réservoir d'eau disposé pour baigner une partie de la cage.

18. Dispositif selon l'une des revendications 1 à 18, **caractérisé en ce que** la lampe (10) , avec laquelle il est destiné à être utilisé, est une lampe à vapeur de mercure moyenne pression.

19. Procédé de test de vieillissement d'échantillons **caractérisé en ce qu'**il met en oeuvre un dispositif d'exposition à des rayonnements selon l'une des revendications 1 à 19.

20. Procédé de test de vieillissement d'échantillons, **caractérisé en ce qu'**il comporte des étapes consistant à :
- disposer les échantillons sur une cage (40) porte-échantillons rotative autour d'un axe (H-H) sensiblement horizontal,
- positionner fixement et alimenter au moins une lampe (10) à rayonnement électromagnétique disposée sur ou autour de l'axe (H-H) de rotation horizontal dans la partie centrale de la cage, et
- générer un flux d'air tourbillonnaire autour de l'axe (H-H) de rotation horizontal, le flux d'air tourbillonnaire ayant, au niveau de la périphérie de la cage porte-échantillons (40), essentiellement un mouvement de rotation dont la composante est comprise entre la direction tangentielle et la direction radiale, le flux d'air tourbillonnaire ayant au niveau de la partie centrale de la cage, essentiellement un mouvement de translation dont la composante est sensiblement parallèle à la direction axiale.

## Claims

1. Device for ageing samples being exposed to radiation, having an enclosure (30) and comprising:
- a sample-holder cage (40) rotating about a horizontal axis (H-H); and
- means (15) for firmly positioning and supplying at least an electromagnetic radiation lamp (10) in the central part of the cage (40),
**characterised in that** it comprises an air circulation system capable of generating a flow of swirling air about the axis (H-H) of the cage, the system comprising fixed air passage apertures (31, 32, 33) arranged about the periphery (41) of the rotating cage (40), and an air passage orifice (50) arranged on the axis (H-H) of the cage.

2. Device set forth in claim 1, **characterised in that** the air circulation system comprises fixed air inlet apertures (31-38) evenly arranged around the periphery (41) of the cage (40) and are rotationally symmetrical about the axis, each aperture (31, 32, 33..., 38) being oriented between the radial and the tangential directions to the periphery of the cage, and an air evacuation conduit (50) arranged on the axial extension (H) of the central part of the cage (40), the conduit (50) being oriented in a direction parallel to the axis (H-H).

3. Device set forth in claim 1 or 2, **characterised in that** the cage (40) is rotationally assembled on a hub (50) hollowed out along the axis (H-H), the air flow discharging into the axial cavity of the hub.

4. Device set forth in claim 3, **characterised in that** the air circulation system comprises air suction means (1) arranged on the cavity opening of the hub.

5. Device set forth in one of claims 1 to 4, **characterised in that** the air circulation system is an open circuit.

6. Device set forth in one of claims 1 to 5, **characterised in that** the cylindrical periphery (30) comprises air inlet suction eyes (31-38) substantially arranged around the periphery of the cage (40).

7. Device set forth in claim 6, **characterised in that** the air inlet suction eyes (31-38) around the periphery of the enclosure (30) are oriented in a non-radial direction.

8. Device set forth in claim 7, **characterised in that** each air inlet suction eye (31) has a depth and an angle of tilt in a radial direction so that the radiation of each lamp (10) placed in the central part of the cage (40) does not cross the suction eye (31) and does not directly discharge beyond the enclosure (30).

9. Device set forth in one of claims 1 to 8, comprising means (15) for axially positioning an electromagnetic radiation lamp on the axis (H-H) of the cage.

10. Device set forth in one of claims 1 to 9, comprising means for symmetrically positioning electromagnetic radiation lamps about the axis of the cage.

11. Device set forth in one of claims 1 to 10, **characterised in that** the cage (40) comprises central elements (47') and peripheral elements (47"') extending in the axial direction (H-H) and connected via the radial elements (47"), the central and peripheral elements (47', 47"') extend into a single half-space delimited by the radial elements (47").

12. Device set forth in one of claims 1 to 11, **characterised in that** the cage comprises circular elements (41, 44, 44') with different diameters allowing the samples to be arranged at different angles of exposure.

13. Device set forth in claim 11 or 12, **characterised in that** the central elements of the cage are arranged helically about the axis.

14. Device set forth in one of claims 3 to 13, **characterised in that** the cage (40) is linked to the hub (50) via a disengaging drive mechanism (51, 55, 56, 57) allowing to freely rotate the cage.

15. Device set forth in claim 14, **characterised in that** the mechanism comprises disengaging means rotating under the influence of an excessive axial torque between the cage and the hub.

16. Device set forth in claim 14 or 15, **characterised in that** the mechanism comprises disengaging or uncoupling means translating under the influence of an excessive axial force between the cage and the hub.

17. Device set forth in one of claims 1 to 16, **characterised in that** it comprises a water tank arranged to bath a part of the cage.

18. Device set forth in one of claims 1 to 17, **characterised in that** the lamp (10), with which it is intended to be used, is a mid-pressure mercury vapour lamp.

19. Method for testing age sampling **characterised in that** it implements a radiation exposure device according to claims 1 to 18.

20. Method for testing age sampling **characterised in that** it comprises stages consisting in:
- arranging the samples on a sample-holder cage (40) that rotates about a substantially horizontal axis (H-H);
- firmly positioning and supplying at least an electromagnetic radiation lamp (10) arranged on or about the horizontal axis of rotation (H-H) in the central part of the cage; and
- generating a swirling air flow about the horizontal axis of rotation (H-H), the swirling air flow, in the vicinity of the periphery of the sample-holder cage (40), principally bearing a rotational movement whose component is included between the tangential direction and the radial direction, the swirling air flow, in the vicinity of the central part of the cage, principally bearing a translational movement whose component is substantially parallel to the axial direction.

## Patentansprüche

1. Bestrahlungsvorrichtung zum Testen der Alterung von Proben mit einer Einfassung (30) und mit:
- einem Käfig (40) für die Probenhalterung, der um eine horizontale Achse (H-H) drehbar ist, und
- einer Einrichtung (15) zum festen Positionieren und Versorgen wenigstens einer Lampe (10) mit elektromagnetischer Strahlung im zentralen Teil des Käfigs (40),
**dadurch gekennzeichnet, dass** diese ein Luftzirkulationssystem umfasst, das so ausgelegt ist, dass es einen turbulenten Luftstrom um die Achse (H-H) des Käfigs herum erzeugt, wobei das System festgelegte Luftdurchgangsöffnungen (31, 32, 33), die um den Umfang (41) des drehbaren Käfigs (40) herum angeordnet sind, und eine Luftdurchgangsöffnung (50), die in Richtung der Achse (H-H) des Käfigs angeordnet ist, aufweist.

2. Vorrichtung nach Anspruch 1,
**dadurch gekennzeichnet, dass** das Luftzirkulationssystem festgelegte Luftzutrittsöffnungen (31 bis 38) aufweist, die am Umfang (41) des Käfigs (40) regelmäßig, mit einer Rotationssymmetrie um die Achse herum angeordnet sind, wobei jede Öffnung (31, 32, 33, ... 38) in einer Richtung zwischen der radialen Richtung und der tangentialen Richtung zum Umfang des Käfigs ausgerichtet ist, und einen Luftevakuierungskanal (50) aufweist, der in der axialen Verlängerung (H) des zentralen Teils des Käfigs (40) angeordnet ist, wobei der Kanal (50) in einer parallelen Richtung zur Achse (H-H) ausgerichtet ist.

3. Vorrichtung nach Anspruch 1 oder 2,
**dadurch gekennzeichnet, dass** der Käfig (40) drehbar auf einer axial (H-H) hohlen Nabe (50) montiert ist, wobei der Luftstrom in den axialen Hohlraum der Nabe abströmt.

4. Vorrichtung nach Anspruch 3,
**dadurch gekennzeichnet, dass** das Luftzirkulationssystem eine Luftansaugeinrichtung (1) umfasst, die in der Hohlöffnung der Nabe angeordnet ist.

5. Vorrichtung nach einem der Ansprüche 1 bis 4,
**dadurch gekennzeichnet, dass** das Luftzirkulationssystem ein offener Kreislauf ist.

6. Vorrichtung nach einem der Ansprüche 1 bis 5,
**dadurch gekennzeichnet, dass** die zylindrische Einfassung (30) Lufteinlässe (31 bis 38) aufweist, die im Wesentlichen am Umfang des Käfigs (40) angeordnet sind.

7. Vorrichtung nach Anspruch 6,
**dadurch gekennzeichnet, dass** die am Umfang der Einfassung (30) befindlichen Lufteinlässe (31 bis 38) nicht-radial ausgerichtet sind.

8. Vorrichtung nach Anspruch 7,
**dadurch gekennzeichnet, dass** jeder umfängliche Lufteinlass (31) eine Tiefe, eine Öffnung und einen Neigungswinkel in Bezug zur radialen Richtung aufweist, derart, dass die Strahlung jeder im zentralen Teil des Käfigs (40) positionierten Lampe (10) den Einlass (31) nicht durchtritt und nicht direkt aus der Einfassung (30) entweicht.

9. Vorrichtung nach einem der Ansprüche 1 bis 8, mit einer Einrichtung (15) zum axialen Positionieren einer Lampe (10) für die elektromagnetische Strahlung auf der Achse (H-H) des Käfigs.

10. Vorrichtung nach einem der Ansprüche 1 bis 9, mit Einrichtungen zum symmetrischen Positionieren von elektromagnetischen Strahlungslampen um die Achse des Käfigs herum.

11. Vorrichtung nach einem der Ansprüche 1 bis 10,
**dadurch gekennzeichnet, dass** der Käfig (14) zentrale Elemente (47') und Umfangselemente (47"') umfasst, die sich in axialer Richtung (H-H) erstrecken und durch radiale Elemente (47") verbunden sind, wobei sich die Zentral- und Umfangselemente (47', 47" ') in einem einzigen durch die radialen Elemente (47") begrenzten Halbraum erstrecken.

12. Vorrichtung nach einem der Ansprüche 1 bis 11,
**dadurch gekennzeichnet, dass** der Käfig Ringelemente (41, 44, 44') mit unterschiedlichen Durchmessern umfasst, die es ermöglichen, Proben in unterschiedlichen Winkellagen anzuordnen.

13. Vorrichtung nach Anspruch 11 oder 12,
**dadurch gekennzeichnet, dass** sich die Zentralelemente schraubenförmig um die Achse herum entwickeln.

14. Vorrichtung nach einem der Ansprüche 3 bis 13,
**dadurch gekennzeichnet, dass** der Käfig (40) durch einen ausskuppelbaren Antriebsmechanismus (51, 55, 56, 57) mit der Nabe (50) verbunden ist, der eine freie Drehung des Käfigs ermöglicht.

15. Vorrichtung nach Anspruch 14,
**dadurch gekennzeichnet, dass** der Mechanismus eine Einrichtung zum Auskuppeln bei Drehung unter der Wirkung eines zwischen dem Käfig und der Nabe begrenzten Axialmoments umfasst.

16. Vorrichtung nach Anspruch 14 oder 15,
**dadurch gekennzeichnet, dass** der Mechanismus eine Einrichtung zur translatorisehen Auskopplung oder Trennung unter dem Einfluss einer zwischen dem Käfig und der Nabe begrenzten Axialbeanspruchung umfasst.

17. Vorrichtung nach einem der Ansprüche 1 bis 16,
**dadurch gekennzeichnet, dass** diese ein Wasserreservoir umfasst, das zum Baden eines Teils des Käfigs vorgesehen ist.

18. Vorrichtung nach einem der Ansprüche 1 bis 17,
**dadurch gekennzeichnet, dass** die Lampe (10) mit welcher diese verwendet werden soll, eine Quecksilbermitteldrucklampe ist.

19. Testverfahren für die Alterung von Proben,
**dadurch gekennzeichnet, dass** dieses eine Bestrahlungsvorrichtung nach einem der Ansprüche 1 bis 18 verwendet.

20. Testverfahren zur Alterung von Proben,
**dadurch gekennzeichnet, dass** dieses die folgenden Schritte umfasst:
- Anordnen von Proben an einem Probenhalter-Käfig (40), der um eine im Wesentlichen horizontale Achse (H-H) drehbar ist,
- fest Positionieren und Versorgen wenigstens einer Lampe (10) mit elektromagnetischer Strahlung, die auf der oder um die horizontale Drehachse (H-H) herum im zentralen Teil des Käfigs angeordnet ist, und
- Erzeugen eines turbulenten Luftstroms um die horizontale Drehachse (H-H) herum, wobei der turbulente Luftstrom am Umfang des Probenhalter-Käfigs (40) im Wesentlichen eine Drehbewegung aufweist, deren Komponente zwischen der tangentialen Richtung und der radialen Richtung verläuft, wobei der turbulente Luftstrom im zentralen Teil des Käfigs eine im Wesentlichen translatorische Bewegung aufweist, deren Komponente im Wesentlichen parallel zur axialen Richtung verläuft.
